# EUROPEAN PATENT APPLICATION

(11) **EP 3 050 555 A1**
(43) Date of publication of application: **03.08.2016**
(21) Application number: 14849049.3
(22) Date of filing: 25.09.2014
(51) Int. Cl.: A61K 8/87, A61K 8/35, A61K 8/55, A61Q 3/02

(54) **ARTIFICIAL NAIL COMPOSITION, ARTIFICIAL NAIL, ARTIFICIAL NAIL FORMING METHOD, AND NAIL ART KIT**

(30) Priority: 27.09.2013 JP 2013201917
(71) Applicant: Fujifilm Corporation, Minato-ku Tokyo 106-8620 (JP)
(72) Inventor: ABE Junya, Haibara-gun Shizuoka 421-0396 (JP)
(74) Representative: Hoffmann Eitle
(86) International application number: PCT/JP2014/075371
(87) International publication number: WO 2015/046300

(57) **Abstract**

Provided are an artificial nail composition which has excellent removability and from which an artificial nail having excellent glossiness and adhesiveness is obtained; an artificial nail produced using the artificial nail composition; a method for forming an artificial nail; and a nail art kit.

The artificial nail composition of the invention includes a polyurethane (meth)acrylate having a polycarbonate structure as component A; a binder polymer having a weight average molecular weight of 10,000 or more as component B; and a photopolymerization initiator as component C. It is preferable that the weight average molecular weight of the component A is 1,000 or more, and it is preferable that the artificial nail composition includes the component B at a proportion of 15% by mass or more relative to the total amount of the artificial nail composition.

## Description

### BACKGROUND OF THE INVENTION

### 1. Field of the Invention

The present invention relates to an artificial nail composition, an artificial nail, a method for forming an artificial nail, and a nail art kit.

### 2. Description of the Related Art

In recent years, nail art for applying designs to finger nails or toe nails is becoming more popular, and the technology of forming artificial nails (nail stickers, nail tips and the like) made of synthetic resins on nails (natural nails) is ever evolving. Recently, in particular, nail decorations called artificial nails, which are produced by applying a gel-like curable composition for decoration containing a urethane-based resin and a photopolymerizable monomer on nails, and then curing the curable composition by irradiating the composition with ultraviolet radiation, are attracting more attention for the reason that the finish is clear, the nail decorations are long-lasting and highly adhesive to the nails, and there is no foul odor as in the case of acrylic resins.

Regarding conventional artificial nail compositions, those compositions described in WO97/00664A and JP2007-161715A are known.

### SUMMARY OF THE INVENTION

An object of the invention is to provide an artificial nail composition which has excellent removability and from which an artificial nail having excellent glossiness and adhesiveness is obtained, an artificial nail using the artificial nail composition, a method for forming an artificial nail, and a nail art kit.

The above-described object was achieved by the means described in the following <1> or <16> to <18>. These are disclosed below together with <2> to <15>, which are preferred embodiments.
<1> An artificial nail composition including a polyurethane (meth)acrylate having a polycarbonate structure as component A; a binder polymer having a weight average molecular weight of 10,000 or more as component B; and a photopolymerization initiator as component C.
<2> The artificial nail composition according to <1>, in which the component A is represented by the following Formula 1:

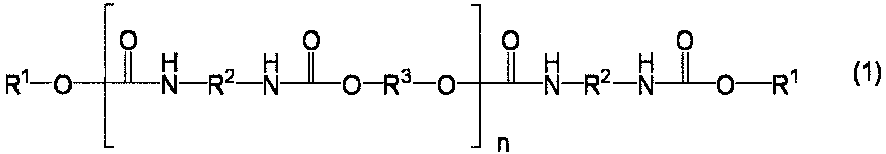

   In Formula 1, R¹'s each independently represent a monovalent group having a (meth)acryloyloxy group at an end; R² and R³ each independently represent a divalent organic group; at least one R³ represents a group represented by the following Formula 2; and n represents an integer of 1 or more:

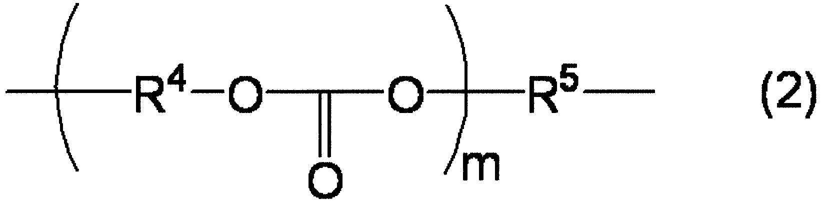

   In Formula 2, R⁴ and R⁵ each independently represent a divalent organic group ;and m represents an integer of 1 or more.
<3> The artificial nail composition according to <1> or <2>, in which the weight average molecular weight of the component A is 1,000 or more (more preferably 2,000 or more, even more preferably 3,000 or more, and particularly preferably 4,000 or more, and preferably 50,000 or less, and more preferably 20,000 or less).
<4> The artificial nail composition according to any one of <1> to <3>, in which the composition includes the component A in an amount of 10% by mass to 70% by mass (more preferably 15% by mass to 55% by mass, and even more preferably 20% by mass to 55% by mass) relative to the total amount of the artificial nail composition.
<5> The artificial nail composition according to any one of <1> to <4>, in which the composition includes the component B in an amount of 15% by mass or more (more preferably 15% by mass to 80% by mass, even more preferably 15% by mass to 70% by mass, and particularly preferably 16% by mass to 60% by mass) relative to the total amount of the artificial nail composition.
<6> The artificial nail composition according to any one of <1> to <5>, in which the component B is selected from the group consisting of an acrylic polymer, a urethane-based polymer, a cellulose-based polymer, an ester-based polymer, an amide-based polymer, a vinyl-based polymer, an ether-based polymer, a styrene-based polymer, a carbonate-based polymer, a urea-based polymer, an ethylene-based polymer and an amine-based polymer (more preferably selected from the group consisting of an ethylene-based polymer, a urethane-based polymer and an acrylic polymer; even more preferably a urethane-based polymer or an acrylic polymer; and particularly preferably a urethane-based polymer).
<7> The artificial nail composition according to any one of <1> to <6>, in which the composition includes an acetophenone compound and/or an acylphosphine oxide compound as the component C.
<8> The artificial nail composition according to any one of <1> to <7>, further including a polymerizable compound as component D.
<9> The artificial nail composition according to <8>, in which the component D includes an ethylenically unsaturated compound.
<10> The artificial nail composition according to <8> or <9>, in which the component D includes a monofunctional ethylenically unsaturated compound.
<11> The artificial nail composition according to any one of <8> to <10>, in which the component D includes at least one compound selected from the group consisting of a (meth)acrylate compound having a hydroxy group, a (meth)acrylate compound having a carboxy group, and (meth)acrylic acid.
<12> The artificial nail composition according to any one of <8> to <11>, in which the component D includes at least one compound selected from the group consisting of hydroxyethyl (meth)acrylate, hydroxypropyl (meth)acrylate, hydroxybutyl (meth)acrylate, a polyalkylene glycol mono(meth)acrylate, and (meth)acrylic acid.
<13> The artificial nail composition according to any one of <8> to <12>, in which the component D includes at least one compound selected from the group consisting of hydroxyethyl (meth)acrylate, hydroxypropyl (meth)acrylate, and (meth)acrylic acid.
<14> The artificial nail composition according to any one of <8> to <13>, in which the component D includes at least hydroxyethyl (meth)acrylate.
<15> The artificial nail composition according to any one of <8> to <14>, in which the content of the component D is 1% by mass to 80% by mass (more preferably 1% by mass to 70% by mass, and even more preferably 1% by mass to 60% by mass) relative to the total mass of the artificial nail composition.
<16> An artificial nail including a layer formed by exposing the artificial nail composition according to any one of <1> to <15> to light.
<17> A method for forming an artificial nail, the method including a step of applying the artificial nail composition according to any one of <1> to <15> on a nail of a human being or an animal, or on another artificial nail, and forming a coating film thereon; and exposing the coating film to light, and thereby forming an artificial nail.
<18> A nail art kit including the artificial nail composition according to any one of <1> to <15>.

According to the invention, an artificial nail composition which has excellent removability and from which an artificial nail having excellent glossiness and adhesiveness is obtained, an artificial nail obtained using the artificial nail composition, a method for forming an artificial nail, and a nail art kit can be provided.

### DESCRIPTION OF THE PREFERRED EMBODIMENTS

Hereinafter, the present invention will be described in detail.

Meanwhile, in the present specification, the description of "xx to yy" represents a value range including xx and yy. Also, the polyurethane (meth)acrylate having a polycarbonate structure or the like that is included in the artificial nail composition of the invention as component A is simply referred to as "component A" or the like.

Furthermore, according to the invention, the units "percent (%) by mass" and "percent (%) by weight" have the same meaning, and the units "parts by mass" and "parts by weight" have the same meaning.

Also, according to the invention, a combination of two or more preferred embodiments is more preferred.

In the present specification, with regard to the description of a group in a compound represented by a formula, in a case in which it is not indicated whether the group is substituted or unsubstituted, and in a case in which the relevant group can further have a substituent, unless particularly stipulated otherwise, the relevant group is intended to include an unsubstituted group as well as a group having a substituent. For example, in regard to an explanation of a formula, when it is described that "R represents an alkyl group, an aryl group or a heterocyclic group", this description means that "R represents an unsubstituted alkyl group, a substituted alkyl group, an unsubstituted aryl group, a substituted aryl group, an unsubstituted heterocyclic group or a substituted heterocyclic group". Also, in the present specification, the term (meth)acryl is intended to include both acryl and methacryl, and the same also applies to the term (meth)acrylate or the like.

A polymer according to the present specification is meant to also include a copolymer and an oligomer.

### (Artificial nail composition)

The artificial nail composition of the invention includes a polyurethane (meth)acrylate having a polycarbonate structure as component A; a binder polymer having a weight average molecular weight of 10,000 or more as component B; and a photopolymerization initiator as component C.

Furthermore, the artificial nail of the invention is an artificial nail having a layer formed from the artificial nail composition of the invention.

The inventors of the present invention conducted a thorough investigation, and as a result, they found that when the artificial nail composition of the invention includes component A to component C, the artificial nail obtainable therefrom has excellent glossiness, removability and adhesiveness.

The mechanism by which these effects are manifested is not clearly known; however, the mechanism is partially speculated to be as follows.

The inventors of the present invention found that a polyurethane (meth)acrylate compound can impart strength as well as flexibility to a cured film; however, in a high temperature and high humidity environment, the cured film cannot maintain strength and flexibility due to the heat-induced plasticization effect of the polymer, degradation caused by moisture absorption, and the like. The inventors conducted a thorough investigation, and as a result, they found that when a polyurethane (meth)acrylate compound is imparted with a polycarbonate structure, due to the mobility of the polycarbonate structure, an interaction effect attributable to the polycarbonate structure occurs while flexibility is maintained, and thus losses in strength and flexibility at high temperature and high humidity can be prevented. In general, artificial nails are degraded when exposed to the living environment; however, it is speculated that high glossiness and adhesiveness can be maintained by preventing the losses in strength and flexibility in the scope of the living environment.

Furthermore, it is speculated that a polycarbonate structure has high affmity to organic solvents that are commonly used when artificial nails are removed, and can impart satisfactory removability.

Moreover, it is speculated that when a polyurethane (meth)acrylate having a polycarbonate structure and a binder polymer having a molecular weight of 10,000 or more are used in combination, the polyurethane (meth)acrylate molecules having a polycarbonate structure are suitably entangled, and high film formability can be imparted, while the adhesiveness can be further increased. Also, it is considered that by using a compound having a high polar group, which is represented by a hydroxy group or a carboxy group, as the polymerizable compound, an effect of further increasing the adhesiveness as a result of an increased interaction between the polar group and the polycarbonate site occurs.

Particularly, a polycarbonate structure can easily induce an interaction with the functional group in the keratin that forms the nail, and also from this point of view, it is speculated that the polycarbonate structure contributes to the high adhesiveness required from an artificial nail composition.

The artificial nail composition of the invention is a composition which forms an artificial nail when cured by light exposure on a nail of a human being or an animal, or on another artificial nail. Furthermore, an artificial nail formed from the artificial nail composition of the invention can be removed by a conventional removal method using an organic solvent.

An artificial nail according to the invention refers to a layer formed on a nail of a human being or an animal for the purpose of a beauty treatment and/or protection. Also, for example, regarding the other artificial nails, a resin substrate having an arbitrary shape intended for a beauty treatment and/or protection of a nail may be mentioned.

Meanwhile, the "nails of a human being and an animal, and other artificial nails" will also be simply referred to as "nails".

The shape of the artificial nail is not particularly limited, and the artificial nail may be formed into a desired shape. For example, the artificial nail may be formed so as to cover the surface of a nail, may be formed on a portion of a nail, or may be formed into a shape larger than a nail using a nail form or the like for the purpose of extension of a nail.

Furthermore, the artificial nail composition of the invention can have the thickness controlled by application. The thickness is not particularly limited as long as the thickness is in the range that can be commonly adopted by an artificial nail; however, from the viewpoints of practical usability and removability, the thickness is preferably in the range of 20 µm to 1,500 µm.

The artificial nail composition of the invention can be suitably used for any of a primer layer, a base layer, a color layer, and/or a top layer of an artificial nail.

Furthermore, an artificial nail layer formed using the artificial nail composition of the invention may be separately provided with a primer layer, a base layer, a color layer, and/or a top layer as an upper layer (surface on the opposite side of the nail) or a lower layer (surface between the artificial nail layer and the nail), for the purpose of providing color, gloss, and adhesion.

The artificial nail composition of the invention is a photocurable artificial nail composition ("artificial nail composition for gel nail").

The photocurable artificial nail composition is an artificial nail composition that can be cured by active light rays. "Active light rays" are radiations that can impart energy for generating an initiation species in an artificial nail composition when the radiations are radiated, and include ultraviolet radiation, visible light and the like. Among them, from the viewpoints of the curing sensitivity and the easy availability of the apparatus, ultraviolet radiation is particularly preferred. Therefore, the photocurable artificial nail composition is preferably an artificial nail composition which can be cured when irradiated with ultraviolet radiation as an active light ray.

The various components that can be used for the artificial nail composition of the invention will be explained below.

### Component A: polyurethane (meth)acrylate having polycarbonate structure

The artificial nail composition of the invention includes a polyurethane (meth)acrylate having a polycarbonate structure (hereinafter, also referred to as "polycarbonate type polyurethane (meth)acrylate") as component A.

The weight average molecular weight of the component A is preferably 1,000 or more, more preferably 2,000 or more, even more preferably 3,000 or more, and particularly preferably 4,000 or more. The upper limit of the weight average molecular weight is not particularly limited; however, the upper limit is preferably 50,000 or less, and more preferably 20,000 or less. When the weight average molecular weight is in the range described above, the resulting artificial nail has an excellent balance between film formability and adhesiveness.

According to the invention, the weight average molecular weight can be determined by measuring the molecular weight by a gel permeation chromatography (GPC) method and calculating the molecular weight relative to polystyrene standards. Specifically, for example, GPC is performed by using HLC-8220GPC (manufactured by Tosoh Corporation); three columns, namely, TSKgeL SuperHZM-H, TSKgeL SuperHZ4000, and TSKgeL SuperHZ2000 (manufactured by Tosoh Corp., 4.6 mm ID x 15 cm), are used; and THF (tetrahydrofuran) is used as an eluent liquid. Furthermore, GPC is performed using an IR detector under the conditions including a sample concentration of 0.35% by mass, a flow rate of 0.35 ml/min, a sample injection amount of 10 µl, and a measurement temperature of 40°C. Also, a calibration curve is produced using 8 samples of "Standard Sample TSK standard, polystyrene" such as "F-40", "F-20", "F-4", "F-1", "A-5000", "A-2500", "A-1000" and "n-propylbenzene" manufactured by Tosoh Corp.

The number of (meth)acrylate groups ((meth)acryloyloxy groups) carried by the component A is preferably 2 to 10, more preferably 2 to 3, and particularly preferably 2, in one molecule. When the number of (meth)acryloyloxy groups in one molecule is in the range described above, the cured film maintains an appropriate crosslinking density, and the cured film has suitable flexibility and excellent adhesiveness and removability.

The component A is preferably a polyurethane (meth)acrylate having a polycarbonate structure represented by the following Formula 1:

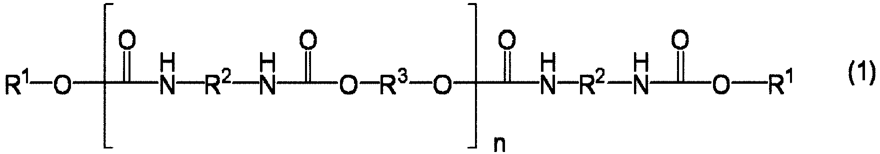

In Formula 1, R¹'s each independently represent a group having a (meth)acryloyloxy group at an end; R² and R³ each independently represent a divalent organic group; at least one R³ is a group represented by the following Formula 2; and n represents an integer of 1 or more:

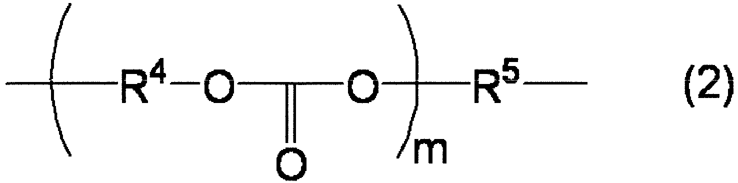

In Formula 2, R⁴ and R⁵ each independently represent a divalent organic group; and m represents an integer of 1 or more.

In Formula 1 and Formula 2, in a case in which there are plural moieties of R¹, R², R³ and R⁴, respectively, they may be identical to or different from each other, and there are no particular limitations.

It is preferable that the compound represented by Formula 1 described above is obtained by subjecting a polyisocyanate compound (a2) (hereinafter, also simply referred to as compound a2) and a polyol compound (a3) (hereinafter, also simply referred to as compound a3) to a polycondensation reaction, and reacting a terminal isocyanate group of the polyurethane compound thus obtained, with a compound having a hydroxy group and a (meth)acryloyloxy group (a1) (hereinafter, also simply referred to as compound a1). Furthermore, a polycarbonate diol is used as at least a portion of the polyol compound (a3).

Meanwhile, the polyisocyanate compound (a2) is preferably a diisocyanate compound, and the polyol compound (a3) is preferably a diol compound. Furthermore, the compound having a hydroxy group and a (meth)acryloyloxy group (a1) is preferably a compound having one hydroxy group and one (meth)acryloyloxy group.

In Formula 1, R² is preferably a urethane bond residue of the polyisocyanate compound (a2), that is, a residue obtained by eliminating an isocyanate group from the polyisocyanate compound (a2), and R³ is preferably a urethane bond residue of the polyol compound (a3), that is, a residue obtained by eliminating a hydroxy group from the polyol compound (a3). Furthermore, R¹ is preferably a urethane bond residue of the compound having a hydroxy group and a (meth)acryloyloxy group (a1), that is, a residue obtained by eliminating a hydroxy group from the compound having a hydroxy group and a (meth)acryloyloxy group (a1).

In Formula 2, it is preferable that R⁴ and R⁵ each independently represent an alkyl group, a group combining an alkylene group and an ether bond, an arylene group, or a group combining an arylene group and an alkylene group. R⁴ and R⁵ may be identical or may be different; however, it is preferable that R⁴ and R⁵ are identical from the viewpoint of synthesis. Furthermore, the R⁴ moieties that exist in a plural number may be each identical, or may be different.

The alkylene group is preferably an alkylene group having 2 to 60 carbon atoms, and more preferably an alkylene group having 2 to 20 carbon atoms. The alkylene group may be any of a linear group, a branched group, or a cyclic group, and may be a combination thereof. Among these, from the viewpoint of flexibility of the artificial nail thus formed, the alkylene group is preferably a linear alkylene group, more preferably a linear alkylene group having 2 to 12 carbon atoms, and even more preferably a linear alkylene group having 3 to 8 carbon atoms.

Preferred examples of the group combining an alkylene group and an ether bond include an alkyleneoxyalkylene group and a poly(alkyleneoxy)alkylene group. The alkylene group is preferably an alkylene group having 2 to 6 carbon atoms; more preferably an alkylene group having 2 to 4 carbon atoms; even more preferably an alkylene group having 2 or 3 carbon atoms; particularly preferably an ethyleneoxyethylene group, a poly(ethyleneoxy)ethylene group, a propyleneoxypropylene group, or a poly(propyleneoxy)propylene group; and most preferably an ethyleneoxyethylene group or a poly(ethyleneoxy)ethylene group.

Examples of the arylene group include a phenylene group and a naphthalenediyl group, and examples of the group combining an arylene group and an alkylene group include -CH₂-Ph-CH₂- (where Ph represents a phenylene group).

In Formula 2, m represents an integer of 1 or more, preferably an integer from 2 to 10, and more preferably an integer from 4 to 8.

The compound having a hydroxy group and a (meth)acryloyloxy group (a1) is preferably a hydroxyalkyl (meth)acrylate or a poly(alkylene glycol) mono(meth)acrylate. The hydroxyalkyl (meth)acrylate is preferably one containing an alkyl group having 2 to 12 carbon atoms, more preferably an alkyl group having 2 to 8 carbon atoms, even more preferably an alkyl group having 2 to 6 carbon atoms, and particularly preferably an alkyl group having 2 to 4 carbon atoms. Furthermore, the alkylene glycol of the poly(alkylene glycol) mono(meth)acrylate is preferably an alkylene glycol having 2 to 6 carbon atoms, more preferably an alkylene glycol having 2 to 4 carbon atoms, and even more preferably ethylene glycol or propylene glycol. Meanwhile, the poly(alkylene glycol) mono(meth)acrylate may contain a single kind of alkylene glycol, or may contain two or more kinds of alkylene glycols.

Preferred examples of the compound having a hydroxy group and a (meth)acryloyloxy group (a1) include hydroxyalkyl (meth)acrylates such as 2-hydroxyethyl (meth)acrylate, 2-hydroxypropyl (meth)acrylate, 3-hydroxypropyl (meth)acrylate, and 4-hydroxybutyl (meth)acrylate; polyethylene glycol mono(meth)acrylate; polypropylene glycol mono(meth)acrylate; poly(ethylene glycol/propylene glycol)-mono(meth)acrylate; polyethylene glycol/polypropylene glycol-mono(meth)acrylate; poly(ethylene glycol/tetramethylene glycol)-mono(meth)acrylate; poly(propylene glycol/tetramethylene glycol)-mono(meth)acrylate; and polypropylene glycol/polybutylene glycol-mono(meth)acrylate. These compounds may be used singly, or in combination of plural kinds thereof.

Regarding the compound having a hydroxy group and a (meth)acryloyloxy group (a1), a commercially available product may be used. Examples thereof include, but are not limited to, BLENMER (registered trademark) series, including BLEMMER E, BLEMMER PE-90, BLEMMER PE-200, BLEMMER PE-350, BLEMMER P, BLEMMER PP-1000, BLEMMER PP-500, BLEMMER PP-800, BLEMMER 50PEP-300, BLEMMER 70PEP-350 B, BLEMMER 55PET-800, BLEMMER PPT series, BLEMMER 10PPB-500 B, BLEMMER AE-90, BLEMMER AE-200, BLEMMER AE-400, BLEMMER AP-150, BLEMMER AP-400, BLEMMER AP-550, BLEMMER PME-100, BLEMMER PME-200, BLEMMER PME-400, BLEMMER PME-1000, BLEMMER 50POEP-800 B, BLEMMER PLE-200, BLEMMER PSE-400, BLEMMER PSE-1300, BLEMMER PAE-50, BLEMMER PAE-100, BLEMMER 43PAPE-600 B, BLEMMER AME-400, BLEMMER ALE series, BLEMMER ANP-300, BLEMMER 75ANP-600, BLEMMER AAE-50, and BLEMMER AAE-300 (all manufactured by NOF Corporation.).

Examples of the polyisocyanate compound (a2) that can be used include known polyisocyanate compounds (for example, methylene diisocyanates (hexamethylene diisocyanate and trimethylhexamethylene diisocyanate), phenylene diisocyanate, tolylene diisocyanate, diphenylmethane diisocyanate, tolidine diisocyanate, naphthalene diisocyanate, triphenylmethane triisocyanate, tris(phenyl isocyanate) thiophosphate, phenylene diisocyanate, lysine diisocyanate, xylene diisocyanate, bis(isocyanatomethyl)cyclohexane, dicyclohexylmethane diisocyanate, isopropylidene bis(cyclohexyl isocyanate), isophorone diisocyanate, dianisidine diisocyanate, and diphenyl ether diisocyanate). Among them, the polyisocyanate compound (a2) is preferably a diisocyanate compound, and preferred examples thereof include hexamethylene diisocyanate, tolylene diisocyanate, isophorone diisocyanate, and diphenylmethane diisocyanate. These compounds may be used singly, or plural kinds thereof may be used in combination.

Regarding the polyol compound (a3), known polyol compounds can be used. Examples of the polyol compounds include an alkylene diol, an aromatic diol, a polyether diol, and a polyester diol.

The alkylene diol is preferably an alkylene diol having 2 to 20 carbon atoms, more preferably an alkylene diol having 2 to 16 carbon atoms, and even more preferably an alkylene diol having 2 to 8 carbon atoms. Meanwhile, the alkylene group may be any of a linear group, a branched group, a cyclic group, or a combination thereof.

The aromatic diol is not particularly limited as long as it is a diol compound having an aromatic group in the molecule, and the aromatic diol preferably has 6 to 30 carbon atoms, and more preferably 6 to 20 carbon atoms.

The polyether diol is preferably a poly(oxyalkylene) glycol, and the alkylene group is preferably a group having 2 to 6 carbon atoms, and more preferably 2 to 4 carbon atoms.

Examples of the alkylene diol include butanediol and hexanediol.

Examples of the aromatic diol include bisphenol A, fluorene dimethanol, xylylene glycol, and resorcine.

Examples of the polyether diol include polyethylene glycol and polypropylene glycol.

Examples of the polyester diol include polyethylene glycol terephthalate.

Among these, an alkylenediol, a polyether diol, and a polyester diol are preferred, and an alkylenediol or a polyether diol is more preferred, a polyether diol is more preferred, and polyethylene glycol and polypropylene glycol are particularly preferred.

These compounds may be used singly, or in combination of plural kinds thereof.

At least one of the polyol compound (a3) is a polycarbonate diol, and is preferably a diol compound represented by the following Formula 2': 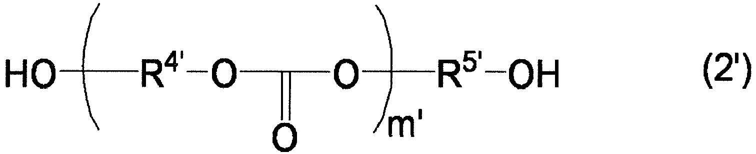 (in Formula 2', R^{4'}, R^{5'} and m' have the same meanings as R⁴, R⁵ and m of Formula 2, respectively, and preferred ranges thereof are also the same).

Specific examples of the compound represented by Formula 2' are described below; however, the invention is not intended to be limited to these specific examples. In the table, the symbol * represents a linking moiety, and m' in Formula 2' has an average value for satisfying the weight average molecular weight indicated in the table. Meanwhile, a-11 to a-13 each have a pentylene group and a hexylene group at 1:1 (molar ratio).

| Compound | R^{4'}= R^{5'} | | Mw |
|---|---|---|---|
| a-1 | 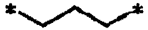 | | 1,600 |
| a-2 | 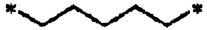 | | 1,700 |
| a-3 | 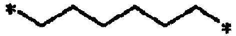 | | 1,800 |
| a-4 | 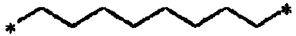 | | 1,400 |
| a-5 | 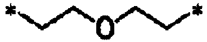 | | 1,300 |
| a-6 | 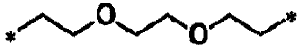 | | 1,200 |
| a-7 | 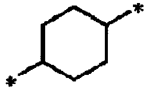 | | 1,500 |
| a-8 | 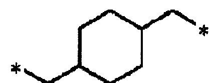 | | 2,000 |
| a-9 | 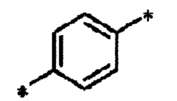 | | 2,400 |
| a-10 | 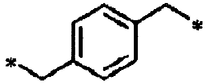 | | 1,500 |
| a-11 | 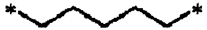 | 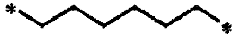 | 2,000 |
| a-12 | 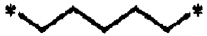 | 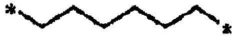 | 500 |
| a-13 | 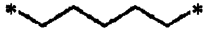 | 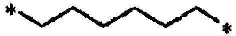 | 3,000 |
| a-14 | 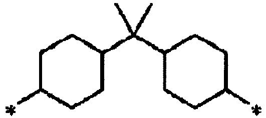 | | 2,000 |

Specific examples of the polycarbonate type polyurethane (meth)acrylate represented by Formula 1 are described below; however, the invention is not intended to be limited to these specific examples. Additionally, compound a1, compound a2, and compound a3 used for the synthesis are shown. n in Formula 1 has an average value for satisfying the weight molecular weight indicated in the table.

| | Compound (a1) | Compound (a2) | Compound (a3) | | | Mw |
|---|---|---|---|---|---|---|
| | | | Compound represented by Formula (2') | Other | Weight ratio | |
| A-1 | 2-Hydroxyethyl acrylate | Isophorone diisocyanate | a-11 | - | - | 5,000 |
| A-2 | 2-Hydroxyethyl acrylate | Tolylene diisocyanate | a-11 | - | - | 5,000 |
| A-3 | 2-Hydroxyethyl acrylate | Hexamethylene diisocyanate | a-11 | - | - | 5,500 |
| A-4 | 2-Hydroxyethyl acrylate | Diphenylmethane diisocyanate | a-11 | - | - | 4,800 |
| A-5 | 2-Hydroxyethyl acrylate | Isophorone diisocyanate | a-11 | Polyethylene glycol (n=2) | 1:1 | 5,000 |
| A-6 | 2-Hydroxyethyl acrylate | Tolylene diisocyanate | a-11 | Polyethylene glycol (n=2) | 1:1 | 5,200 |
| A-7 | 2-Hydroxyethyl acrylate | Hexamethylene diisocyanate | a-11 | Polyethylene glycol (n=2) | 1:1 | 5,300 |
| A-8 | 2-Hydroxyethyl acrylate | Diphenylmethane diisocyanate | a-11 | Polyethylene glycol (n=2) | 1:1 | 5,400 |
| A-9 | 2-Hydroxyethyl acrylate | Isophorone diisocyanate | a-11 | - | - | 5,000 |
| A-10 | 2-Hydroxyethyl acrylate | Isophorone diisocyanate | a-12 | - | - | 5,000 |
| A-11 | 2-Hydroxyethyl acrylate | Isophorone diisocyanate | a-13 | - | - | 5,000 |
| A-12 | 2-Hydroxyethyl acrylate | Isophorone diisocyanate | a-1 | - | - | 5,100 |
| A-13 | 2-Hydroxyethyl acrylate | Isophorone diisocyanate | a-8 | - | - | 5,200 |
| A-14 | 2-Hydroxyethyl acrylate | Isophorone diisocyanate | a-9 | - | - | 5,600 |
| A-15 | 2-Hydroxyethyl acrylate | Isophorone diisocyanate | a-14 | - | - | 5,400 |
| A-16 | 2-Hydroxyethyl acrylate | Isophorone diisocyanate | a-11 | - | - | 8,000 |
| A-17 | 2-Hydroxyethyl acrylate | Isophorone diisocyanate | a-11 | - | - | 20,000 |
| A-18 | 2-Hydroxyethyl acrylate | Isophorone diisocyanate | a-11 | - | - | 60,000 |
| A-19 | 2-Hydroxyethyl methacrylate | Isophorone diisocyanate | a-11 | - | - | 4,800 |
| A-20 | 2-Hydroxyethyl methacrylate | Isophorone diisocyanate | a-12 | - | - | 5,600 |
| A-21 | 2-Hydroxyethyl methacrylate | Isophorone diisocyanate | a-13 | - | - | 5,200 |

Component A may be used singly, or two or more kinds thereof may be used in combination.

The artificial nail composition of the invention preferably includes the component A at a proportion of 10% by mass to 70% by mass, more preferably 15% by mass to 55% by mass, and particularly preferably 20% by mass to 55% by mass, relative to the total amount of the artificial nail composition.

Meanwhile, in a case in which two or more kinds of the component A are used in combination, it is preferable that the total amount is adjusted to the content described above.

Component B: Binder polymer having weight average molecular weight of 10,000 or more

The artificial nail composition of the invention includes a binder polymer having a weight average molecular weight of 10,000 or more as component B. The binder polymer imparts film formability to the artificial nail composition. When the artificial nail composition includes particularly a binder polymer having a weight average molecular weight of 10,000 or more, the interaction between the binder polymer and the polycarbonate type polyurethane (meth)acrylate of the component A is increased, which is suitable.

The upper limit of the weight average molecular weight of the component B is not particularly limited; however, weight average molecular weight is preferably 1,000,000 or less, more preferably 200,000 or less, and particularly preferably 50,000 or less. When the weight average molecular weight is in this molecular weight range, the artificial nail composition has excellent handleability, and the artificial nail formed therefrom has excellent flexibility.

Meanwhile, the component B is a polymer component excluding the component A, and is a polymer component excluding a polyurethane (meth)acrylate having a polycarbonate structure.

The polymer structure for the component B is not particularly limited, and may be any desirable polymer structure. Examples thereof include the polymer structures of an acrylic polymer, a urethane-based polymer, a cellulose-based polymer, an ester-based polymer, an amide-based polymer, a vinyl-based polymer, an ether-based polymer, a styrene-based polymer, a carbonate-based polymer, a urea-based polymer, an ethylene-based polymer, and an amine-based polymer.

From the viewpoints of film formability, handleability (viscousness), and the interaction with the component A, the component B is preferably an ethylene-based polymer (particularly, a polyethyleneimine-based polymer), a urethane-based polymer, or an acrylic polymer; more preferably a urethane-based polymer or an acrylic polymer; and most preferably a urethane-based polymer.

Furthermore, from the viewpoint of imparting water resistance, it is preferable that the component B itself is non-water-soluble. Here, being non-water-soluble means that the component B maintains a heterogeneous appearance without causing mixing or clouding in the same capacity of water under the conditions of 1 atmosphere and 20°C.

Regarding the acrylic polymer, any polymer obtainable by polymerizing a known acrylic acid derivative (for example, an acrylic acid ester such as methyl acrylate or ethyl acrylate, acrylamide, or an acrylic acid amide such as acryloylmorpholine) or a methacrylic acid derivative (for example, a methacrylic acid ester such as methyl methacrylate or ethyl methacrylate, methacrylamide, or a methacrylic acid amide such as methacrylic acid isopropylamide), can all be suitably used. However, the invention is not intended to be limited to these examples.

Regarding the urethane-based polymer, any polyurethane formed from a known polyisocyanate compound (for example, tolylene diisocyanate or isophorone diisocyanate) and a known polyol compound (for example, an alkylenediol such as butanediol or hexanediol; an arylenediol such as dihydroxybenzene or bisphenol A; a polyether diol such as polyethylene glycol or polypropylene glycol; a polyester diol such as polyethylene glycol terephthalate; or a polycarbonate diol such as polyethylene glycol carbonate), can all be suitably used. However, the invention is not intended to be limited to these examples.

Regarding the cellulose-based polymer, any known cellulose such as carboxymethyl cellulose, nitrocellulose, or triacetyl cellulose can be suitably used. However, the invention is not intended to be limited to these examples.

Regarding the ester-based polymer, any polyester formed from a known polycarboxylic acid compound (for example, succinic acid, adipic acid or phthalic acid) and a known polyol compound can all be suitably used. Furthermore, a polyester formed from a hydroxycarboxylic acid compound such as polylactic acid can also be suitably used. However, the invention is not intended to be limited to these examples.

Regarding the amide-based polymer, any polyamide formed from a known polycarboxylic acid compound and a known polyamine compound (for example, ethylenediamine or phenylenediamine) can all be suitably used. Furthermore, a polyamino acid, which is a protein formed from amino acids, can also be suitably used. However, the invention is not intended to be limited to these examples.

Regarding the vinyl-based polymer, a vinyl-based polymer obtainable by polymerizing a known vinyl compound (for example, vinyl acetate, vinyl chloride, or butadiene) can all be suitably used.

Regarding the ether-based polymer, any polyether (for example, polyethylene glycol or polypropylene glycol) formed from a known polyol compound can all be suitably used. However, the invention is not intended to be limited to these examples.

Regarding the styrene-based polymer, any polystyrene formed from a known styrene compound (for example, styrene, 4-carboxystyrene, or 4-acetoxystyrene) can all be suitably used. However, the invention is not intended to be limited to these examples.

Regarding the carbonate-based polymer, any polycarbonate formed from a known carbonic acid derivative (for example, phosgene, dimethyl carbonate, or diethyl carbonate) and a known polyol compound can all be suitably used. However, the invention is not intended to be limited to these examples.

Regarding the urea-based polymer, any polyurea formed from a known polyisocyanate compound and a known polyamine compound can all be suitably used.

Examples of the amine-based polymer include known polyamines.

Furthermore, the component B may also be a copolymerized polymer such as a styrene-acrylic copolymer.

According to the invention, it is preferable that the component B does not contain a polymerizable group. When the component B does not contain a polymerizable group, the component B can be prevented from affecting the flexibility manifested by the component A to an extent more than needed, and altering the adhesiveness.

The component B may be used singly or in combination of two or more kinds thereof.

The content of the component B in the artificial nail composition of the invention is not particularly limited; however, the content is preferably 15% by mass to 80% by mass, more preferably 15% by mass to 70% by mass, and even more preferably 16% by mass to 60% by mass, relative to the total mass of the artificial nail composition. When the content is in the range described above, the artificial has superior adhesiveness and gloss, and also, the artificial nail composition has excellent coatability on the nails.

Furthermore, the content of the component B with respect to the component A is such that the content of the component B is preferably 20 parts by mass to 800 parts by mass, more preferably 30 parts by mass to 450 parts by mass, and even more preferably 30 parts by mass to 300 parts by mass, relative to 100 parts by mass of the content of the component A.

Meanwhile, in a case in which two or more kinds of the component B are used in combination, it is preferable that the total amount is adjusted to the content described above.

### Component C: Photopolymerization initiator

The artificial nail composition of the invention includes a photopolymerization initiator as component C. When the artificial nail composition includes the component C, the artificial nail composition of the invention can be suitably used as a photocurable artificial nail composition.

Examples of the photopolymerization initiator include a radical photopolymerization initiator and a cationic photopolymerization initiator; however, it is more preferable that a radical photopolymerization initiator is included.

Regarding the photopolymerization initiator, any known photopolymerization initiator can be used. The photopolymerization initiator that can be used for the invention may be used singly, or in combination of two or more kinds thereof. Also, it is acceptable to use a radical photopolymerization initiator and a cationic photopolymerization initiator in combination.

The photopolymerization initiator that can be used for the invention is a compound which absorbs light and produces a polymerization initiation species. Examples of the light include γ-radiation, β-radiation, an electron beam, ultraviolet radiation, visible light, and infrared radiation.

Specific preferred examples of the photopolymerization initiator that can be used for the invention include the following compounds. However, the invention is not intended to be limited to these examples.

Acetophenone compounds (for example, 1-hydroxycyclohexyl phenyl ketone, acetophenone, 2,2-dimethoxy-2-phenylacetophenone, 2,2-diethoxyacetophenone, 2-hydroxy-2-methylpropiophenone, 4'-isopropyl-2-hydroxy-2-methylpropiophenone, 2-methyl-1-[4-(methylthio)phenyl]-2-morpholino-1-propane, 2-benzyl-2-dimethylamino-1-(4-morpholinophenyl)-butan-1-one, benzoin, benzoin methyl ether, benzoin ethyl ether, and benzoin propyl ether); benzophenone compounds (for example, benzophenone, and 4,4'-bis(dimethylamino)benzophenone, 3,3-dimethyl-4-methoxy-benzophenone); anthraquinone compounds (for example, anthraquinone, 2-methylanthraquinone, 2-ethylanthraquinone, and tert-butylanthraquinone); thioxanthone compounds (for example, 2-chlorothioxanthone, diethylthioxanthone, isopropylthioxanthone, and diisopropylthioxanthone); trihaloalkyl compounds (for example, 2,4,6-(trichloromethyl)triazine, 2,4-trichloromethyl-6-(4-methoxyphenyl)triazine, and tribromomethylphenylsulfone); lophine dimer compounds (for example, 2-(o-chlorophenyl)-4,5-diphenylimidazolyl dimer); acridine compounds (for example, 9-phenylacridine, 1,7-bis(9-acridinyl)heptane, 1,5-bis(9-acridinyl)pentane, and 1,3-bis(9-acridinyl)propane); acylphosphine oxide compounds (for example, trimethylbenzoyldiphenylphosphine oxide and bis(2,4,6-trimethylbenzoyl)phenylphosphine oxide); metallocene compounds (for example, biscyclopentadienylbis(difluoro-pyrryl-phenyl)titanium); onium salts (for example, bis(4-t-butylphenyl)iodonium tosylate and triphenylsulfonium tosylate); and oxime ester compounds (for example, 1-(4-phenylthiophenyl)-1,2-octanedione-2-(O-benzoyl oxime) and 1-[9-ethyl-6-(2-methylbenzoyl)-9H-carbazol-3-yl]-ethanone-1-(O-acetyl oxime)).

Among them, the component C is preferably a photopolymerization initiator selected from the group consisting of an acetophenone compound, an acylphosphine oxide compound, a metallocene compound, and a lophine dimer compound, and is particularly preferably a photopolymerization initiator selected from the group consisting of an acetophenone compound and an acylphosphine oxide compound, from the viewpoint of curability.

According to the invention, the component C may be used singly, or two or more kinds thereof may be used in combination.

The content of the component C in the artificial nail composition of the invention is preferably 0.01% by mass to 20% by mass, more preferably 0.1% by mass to 15% by mass, and even more preferably 0.5% by mass to 12% by mass, relative to the total mass of the artificial composition. When the content is in the range described above, the artificial nail thus obtainable has excellent adhesiveness, gloss and water resistance.

Meanwhile, in a case in which two or more kinds of the component C are used in combination, it is preferable that the total amount is adjusted to the content described above.

### Component D: Polymerizable compound

It is preferable that the artificial nail composition of the invention includes a polymerizable compound as component D. When the artificial nail composition includes the component D, the artificial nail composition of the invention can be suitably used as a photocurable artificial nail composition.

The component D may be a radical polymerizable compound or a cationic polymerizable compound; however, the component D is preferably a radical polymerizable compound.

Furthermore, the component D is preferably an ethylenically unsaturated compound.

The radical polymerizable compound is a compound having an ethylenically unsaturated bond capable of radical polymerization (ethylenically unsaturated compound), and any compound may be used as long as it is a compound having at least one ethylenically unsaturated bond capable of radical polymerization in the molecule, while the radical polymerizable compound is meant to include compounds having chemical forms such as a monomer and an oligomer. The radical polymerizable compound may be used singly, or two or more kinds thereof may be used in combination at any arbitrary ratio in order to enhance intended characteristics. At this time, it is more preferable to use at least one monofunctional compound (monofunctional ethylenically unsaturated compound), from the viewpoint of controlling the performance such as reactivity and physical properties.

Furthermore, the component D is a compound other than the component A or the component B, and the component D is preferably a compound having a molecular weight of less than 3,000, and more preferably a compound having a molecular weight of less than 1,000.

Examples of the polymerizable compound having an ethylenically unsaturated bond capable of radical polymerization include radical polymerizable compounds such as unsaturated carboxylic acids such as acrylic acid, methacrylic acid, itaconic acid, crotonic acid, isocrotonic acid, and maleic acid, and salts thereof; anhydrides having ethylenically unsaturated groups, acrylonitrile, styrene, various unsaturated polyesters, unsaturated polyethers, unsaturated polyamides, and oligomers of unsaturated urethanes.

Specific examples include (meth)acrylic acid derivatives such as 2-ethylhexyl (meth)acrylate, 2-hydroxyethyl (meth)acrylate, butoxyethyl (meth)acrylate, carbitol (meth)acrylate, cyclohexyl (meth)acrylate, benzyl (meth)acrylate, methyl (meth)acrylate, ethyl (meth)acrylate, n-propyl (meth)acrylate, isopropyl (meth)acrylate, n-butyl (meth)acrylate, allyl (meth)acrylate, glycidyl (meth)acrylate, N-methylol (meth)acrylamide, diacetone (meth)acrylamide, and epoxy (meth)acrylate; and derivatives of allyl compounds such as allyl glycidyl ether, diallyl phthalate, and triallyl trimellitate. More specifically, commercially available products or radical polymerizable or crosslinkable monomers and oligomers known in the industry described in Yamashita, Shinzo, ed., "Handbook of Crosslinking Agent" (1981, Taiseisha, Ltd.); Kato, Kiyomi, ed., " UV·EB Curing Handbook (Raw materials section)" (1985, Kobunshi Kankokai); RadTech Japan, ed., "Applications and Markets of UV·EB Curing Technology", p. 79 (1989, CMC Publishing, Inc.); Takiyama, Eiichiro, "Handbook of Polyester Resins", (1988, Nikkan Kogyo Shimbun, Ltd.); and the like, can be used.

Regarding the component D, from the viewpoints of the strength and flexibility exhibited when the component D is used in combination with the component A, and of the removability, adhesiveness and gloss of the artificial nails thus obtainable, it is preferable that the component D includes an ethylenically unsaturated compound having a hydroxyl group, an ethylenically unsaturated compound having a carboxy group, or an ethylenically unsaturated compound having a (poly)alkyleneoxy group; more preferably a (meth)acrylate compound having a hydroxy group, a (meth)acrylate compound having a carboxy group, or (meth)acrylic acid; even more preferably a (meth)acrylate compound having a hydroxy group or (meth)acrylic acid; particularly preferably a (meth)acrylate compound having a hydroxy group; and most preferably a monofunctional (meth)acrylate compound having a hydroxy group.

Preferred examples of the (meth)acrylate compound having a hydroxy group include hydroxyalkyl (meth)acrylates such as 2-hydroxyethyl (meth)acrylate, 2-hydroxypropyl (meth)acrylate, 3-hydroxypropyl (meth)acrylate, 2,3-dihydroxypropyl (meth)acrylate, and 4-hydroxybutyl (meth)acrylate; polyethylene glycol mono(meth)acrylate; polypropylene glycol mono(meth)acrylate; poly(ethylene glycol/propylene glycol)-mono(meth)acrylate; polyethylene glycol/polypropylene glycol-mono(meth)acrylate; poly(ethylene glycol/tetramethylene glycol)-mono(meth)acrylate; poly(propylene glycol/tetramethylene glycol)-mono(meth)acrylate; and propylene glycol/polybutylene glycol-mono(meth)acrylate.

Preferred examples of the (meth)acrylate compound having a (poly)alkyleneoxy group include methoxypolyethylene glycol-(meth)acrylate, octoxypolyethylene glycol-polypropylene glycol (meth)acrylate, lauroxypolyethylene glycol-(meth)acrylate, stearoxypolyethylene glycol-(meth)acrylate, phenoxypolyethylene glycol-(meth)acrylate, phenoxypolyethylene glycol-polypropylene glycol (meth)acrylate, nonylphenoxy-polyethylene glycol-(meth)acrylate, nonylphenoxy-polypropylene glycol (meth)acrylate, and nonylphenoxy-poly(ethylene glycol-propylene glycol)-(meth)acrylate.

Regarding the (meth)acrylate compound having a hydroxy group and the

(meth)acrylate compound having a (poly)alkyleneoxy group, commercially available products can be used. Representative examples thereof include BLEMMER (registered trademark) series, including BLEMMER E, BLEMMER PE-90, BLEMMER PE-200, BLEMMER PE-350, BLEMMER P, BLEMMER PP-1000, BLEMMER PP-500, BLEMMER PP-800, BLEMMER 50PEP-300, BLEMMER 70PEP-350 B, BLEMMER 55PET-800, BLEMMER PPT series, BLEMMER 10PPT-500 B, BLEMMER AE-90, BLEMMER AE-200, BLEMMER AE-400, BLEMMER AP-150, BLEMMER AP-400, BLEMMER AP-550, BLEMMER PME-100, BLEMMER PME-200, BLEMMER PME-400, BLEMMER PME-1000, BLEMMER 50POEP-800 B, BLEMMER PLE-200, BLEMMER PSE-400, BLEMMER PSE-1300, BLEMMER PAE-50, BLEMMER PAE-100, BLEMMER 43 PAPE-600 B, BLEMMER AME-400, BLEMMER ALE series, BLEMMER ANP-300, BLEMMER 75 ANP-600, BLEMMER AAE-50, and BLEMMER AAE-300 (all manufactured by NOF Corporation.).

Examples of the (meth)acrylate compound having a carboxy group include 2-carboxyethyl (meth)acrylate, 2-(meth)acryloyloxyethylmaleic acid, 2-(meth)acryloyloxyethylsuccinic acid, 2-(meth)acryloyloxyethylphthalic acid, 2-(meth)acryloyloxyethylhexahydrophthalic acid, 1-methyl-2-(meth)acryloyloxypropylphthalic acid, 2-(meth)acryloyloxyethyl-2-hydroxyethylphthalic acid, 1-methyl-2-(meth)acryloyloxyethyl-2-hydroxypropylphthalic acid, and 2-(meth)acryloyloxyethyl-2-hydroxy-3-chloropropylphthalic acid.

In a case in which an ethylenically unsaturated compound having a hydroxy group is used, the number of hydroxy groups in the relevant compound is preferably 1 to 10, more preferably 1 to 5, even more preferably 1 to 3, and particularly preferably 1.

Furthermore, in a case in which an ethylenically unsaturated compound having a (poly)alkyleneoxy group is used, the number of repeating units of the alkyleneoxy group in the relevant compound is preferably 1 to 25, more preferably 1 to 15, and even more preferably 1 to 10.

Among these, the component D is preferably hydroxyethyl (meth)acrylate, hydroxypropyl (meth)acrylate, hydroxybutyl (meth)acrylate, a polyalkylene glycol mono(meth)acrylate, or (meth)acrylic acid; more preferably hydroxyethyl (meth)acrylate, hydroxypropyl (meth)acrylate, or (meth)acrylic acid; even more preferably hydroxyethyl (meth)acrylate or (meth)acrylic acid; and particularly preferably hydroxyethyl (meth)acrylate.

The component D may be included singly or in combination of two or more kinds thereof.

The content of the component D in the artificial nail composition of the invention is not particularly limited; however, the content is preferably 1 % by mass to 80% by mass, more preferably 1% by mass to 70% by mass, and particularly preferably 1% by mass to 60% by mass, relative to the total mass of the artificial nail composition. When the content is in the range described above, the artificial nail obtainable therefrom has excellent removability and water resistance, and the artificial nail composition has excellent coatability on the nails.

Furthermore, in a case in which a monofunctional polymerizable compound and a polyfunctional polymerizable compound are used in combination as the component D, it is preferable that the content of the polyfunctional polymerizable compound is smaller than the content of the monofunctional polymerizable compound. According to the aforementioned embodiment, the artificial nail obtainable therefrom has excellent flexibility. In a case in which the content of the monofunctional polymerizable compound is designated as 100 parts by mass, the content of the polyfunctional polymerizable compound is preferably 100 parts by mass or less, more preferably 50 parts by mass or less, even more preferably 30 parts by mass or less, and particularly preferably 10 parts by mass or less, and it is most preferable that no polyfunctional polymerizable compound is included.

Meanwhile, in a case in which two or more kinds of the component D are used in combination, it is preferable that the total amount is adjusted to the content described above.

### Component E: Colorant

The artificial nail composition of the invention can also be suitably used for a color layer or a top layer. In order to use the artificial nail composition for a color layer or a top layer, the artificial nail composition may include a colorant or a brightening agent. Examples of such a colorant include a dye, an inorganic coloring pigment, an organic coloring pigment, an organic color, a pearl pigment, and a lame coloring material.

More specifically, preferred examples of the pigment include organic coloring pigments such as condensed azo-based pigments, diketopyrrolopyrrole-based pigments, perylene-based pigments, quinacridone-based pigments, isoindolinone-based pigments, dioxazine-based pigments, and phthalocyanine-based pigments; and inorganic coloring pigments such as titanium-based pigments and carbon black-based pigments.

### <Other components>

The artificial nail composition of the invention can optionally include additive components that can be commonly incorporated into the artificial nail composition, as additional components other than the component A to the component E mentioned above, to the extent that the effects of the invention are not impaired. Examples of such additive components include a anti-foaming agent, a buffering agent, a chelating agent, a dispersant, a dye, a filler, a pigment, an antiseptic agent, a resin powder (for example, poly(meth)acrylic acid), an inorganic powder (for example, silica gel), a thickening agent, a wetting agent, a silicone leveling agent, and a fragrance. However, the other components are not limited to these examples.

The artificial nail composition of the invention may include a solvent from the viewpoint of coatability; however, from the viewpoints of safety and handleability, the content of the solvent is preferably less than 10% by mass, and more preferably less than 5% by mass, relative to the total mass of the artificial nail composition, and it is particularly preferable that the artificial nail composition of the invention does not include a solvent.

Regarding the solvent, any known solvent can all be suitably used. Examples thereof include alcohol-based solvents such as ethanol, isopropanol, ethylene glycol monomethyl ether, ethylene glycol monobutyl ether, propylene glycol monomethyl ether, diethylene glycol monoethyl ether and dipropylene glycol monomethyl ether, and solvents based on acetates thereof; ester-based solvents such as ethyl acetate and butyl acetate; ketone-based solvents such as acetone, methyl ethyl ketone, methyl isobutyl ketone, and cyclohexanone; and ether-based solvents such as tetrahydrofuran and methyl-t-butyl ether. However, the solvent is not limited to these examples.

From the viewpoint of the drying speed, the boiling point of the solvent is preferably 30°C to 130°C, more preferably 35°C to 100°C, even more preferably 40°C to 90°C, and particularly preferably 50°C to 85°C. When the boiling point is in the range described above, the artificial nail composition has excellent dryability and coatability.

The solvent may be included singly or in combination of two or more kinds thereof.

Regarding the solvent, it is preferable that the artificial nail composition includes an alcohol-based solvent and/or a ketone-based solvent, and more preferably an alcohol-based solvent and a ketone-based solvent.

Furthermore, the amounts of incorporation of the various components in the artificial nail composition of the invention are not particularly limited; however, it is preferable that the amounts of incorporation are in the range such that component A / component B / component C / component D / other components = 1 to 80 / 16 to 70 / 0.1 to 15 / 5 to 70, as a mass ratio. When the amounts of incorporation are in the range described above, the artificial nail composition has excellent coatability, and the artificial nail obtainable therefrom has excellent adhesiveness and gloss.

### (Artificial nail)

The artificial nail of the invention is an artificial nail having a layer formed from the artificial nail composition of the invention, and the artificial nail is preferably an artificial nail having a layer formed by exposing the artificial nail composition of the invention to light.

The artificial nail of the invention is suitable as a gel nail.

It is desirable that at least a portion of the artificial nail of the invention is formed from the artificial nail composition of the invention, and the artificial nail may have other layers or structures, or the entirety of the artificial nail may be formed from the artificial nail composition of the invention.

A layer formed from the artificial nail composition of the invention can be suitably used as any of a primer layer, a base layer, a color layer, and/or a top layer in the artificial nail of the invention.

Furthermore, the artificial nail of the invention may have only one layer formed from the artificial nail composition of the invention, or may have two or more such layers.

Also, the thickness of the layer formed from the artificial nail composition of the invention in the artificial nail of the invention is not particularly limited; however, the thickness is preferably 10 µm to 5,000 µm, more preferably 20 µm to 3,500 µm, and even more preferably 20 µm to 2,000 µm.

### (Method for forming artificial nail)

The method for forming an artificial nail of the invention is not particularly limited as long as it is a method of forming an artificial nail using the artificial nail composition of the invention; however, the method is preferably a method including a step of applying the artificial nail composition of the invention on a nail of a human being or an animal, or on another artificial nail, and forming a coating film thereon; and a step of exposing the coating film to light, and forming an artificial nail.

### <Coating step>

It is preferable that the method for forming an artificial nail of the invention includes a step of applying the artificial nail composition of the invention on a nail of a human being or an animal, or on another artificial nail, and forming a coating film thereon (coating step).

The other artificial nail is not particularly limited as long as it is a substrate used for artificial nails, and examples thereof include a resin substrate, an artificial nail formed by a method other than the method for forming an artificial nail of the present invention, and an artificial nail obtained by the method for forming an artificial nail of the invention.

The coating method is not particularly limited, and may be carried out by any known method; however, a preferred method is a method of applying the artificial nail composition using a paint brush or a writing brush. Furthermore, spray coating or inkjet coating may also be carried out.

The thickness of the coating film is also not particularly limited, and the coating thickness may be appropriately adjusted while considering the desired thickness for the artificial nail thus obtainable.

### <Exposure step>

In a case in which the artificial nail is formed by exposure to light, examples of the light used for the exposure include ultraviolet radiation and visible light, and from the viewpoints of the curing sensitivity and easy availability of the apparatus, ultraviolet radiation is particularly preferred.

The means for exposure is not particularly limited, and any known means for exposure can be used. Examples thereof include ultraviolet lamps (UV lamps) such as a mercury lamp and a metal halide lamp; a light emitting diode (LED), and a laser diode (LD).

Among them, a UV lamp or an ultraviolet light emitting diode (UV-LED) is preferred.

The exposure time is not particularly limited; however, the exposure time is preferably 10 seconds to 15 minutes, more preferably 30 seconds to 10 minutes, and even more preferably 0.5 minutes to 7 minutes. Furthermore, the exposure may be performed intermittently or continuously, or may be performed using pulse light, and exposure may be achieved by any arbitrary method.

It is preferable that the method for forming the artificial nail of the invention further includes a step of cleaning or wiping the surface of the artificial nail thus obtained, after the exposure step, from the viewpoints of the gloss or the aesthetic appearance of the artificial nail obtainable thereby, and in a case in which exposure has been performed, from the viewpoint of removing uncured components.

Examples of the cleaning or wiping method include a method of wiping using a wiping material such as a wiping sheet or a sponge wipe, which has been soaked with a solvent such as ethanol; and a method of cleaning with a solvent such as ethanol, or water.

Furthermore, the solvent used for the cleaning or wiping is preferably a solvent that does not dissolve the artificial nail thus obtained.

It is preferable that the method for forming an artificial nail of the invention includes a step of roughening the surface of the nail of a human being or an animal, or the other artificial nail, before the coating step. According to the embodiment described above, the artificial nail obtainable by light exposure (gel nail) has excellent adhesiveness and durability.

The method for roughening the nail surface is not particularly limited, and roughening can be carried out by any known method. For example, a method of performing surface roughening using a nail file such as a file may be preferably used.

Furthermore, the method for forming an artificial nail of the invention may also include other known steps. For example, in a case in which the artificial nail composition includes a solvent, the method may include a drying step. Also, it is acceptable to repeatedly carry out the coating step and the exposure step.

### (Method for removing artificial nail)

The artificial nail composition of the invention can be removed by embrittling the artificial nail using an organic solvent such as acetone, which is a conventional removal liquid.

A specific example of the removal method is described below. That is, the surface and/or the tip of the artificial nail is arbitrarily rubbed with a nail file, and thereby the artificial nail is coarsely scratched to the extent that the layer formed from the artificial nail composition of the invention is partially exposed at the surface layer. Subsequently, a cotton ball soaked with acetone is mounted on the gel nail, this is wrapped with aluminum foil or the like and is left to stand for about 3 to 5 minutes so as to swell the gel nail. Subsequently, this swollen gel nail can be very easily and safely removed using a spatula-shaped or stick-shaped tool, without imposing a burden on the fingertip or the nail.

Meanwhile, it is also possible to perform wiping, pushing and tearing while the artificial nail is immersed in the above-described method, and the artificial nail can be removed more quickly. Also, wiping and detachment may also be accelerated by applying ultrasonic waves, vibration or the like.

At this time, the organic solvent used for the removal is not particularly limited. Also, even if an organic solvent is not used, any liquid having a function of embrittling artificial nails can be suitably used. Specific examples thereof include acidic, neutral or alkaline warm water, paraffin, and aromatic oil.

### (Nail art kit)

The nail art kit of the invention includes the artificial nail composition of the invention. A preferred embodiment of the artificial nail composition of the invention in the nail art kit of the invention is as described above.

Also, the nail art kit of the invention may also include any arbitrary object in addition to the artificial nail composition.

Examples thereof include, but are not limited to, a removal liquid; an artificial nail composition other than the artificial nail composition of the invention for a color layer or a top layer; a nail file such as a file; a writing brush or a paint brush, such as a flat brush, for applying the artificial nail composition; an exposure apparatus such as a UV light, a liquid for wiping or cleaning; a wipe for wiping; a nail brush; a dust brush; a nail form used for extending the nail; decorative stones made of acryl, glass, metals or natural stones; a nail seal; a decorative powder such as glitters or holograms; a cutter; a spatula; a stick; and separators for separating the distances between fingers in order to prevent contact between nails.

### EXAMPLES

Hereinafter, the present invention will be more specifically described by way of Examples, but the invention is not intended to be limited to the forms of these Examples. Additionally, unless particularly stated otherwise, the units "parts" and "percent (%)" are on a mass basis.

Furthermore, A-1 to A-21 described in the Examples refer to the same polyurethane (meth)acrylate having a polycarbonate structure as A-1 to A-21 described above.

### <Synthesis Example>

19.9 g of isophorone diisocyanate (manufactured by Evonik Degussa GmbH), 182 g (0.091 moles) of a polyol having an average molecular weight of 2,000 (polyalkylene carbonate diol, manufactured by Asahi Kasei Chemicals Corp., DURANOL T-5652), and 600 g of toluene were introduced into a four-necked flask equipped with a thermometer, a stirrer, a water-cooled condenser, and a nitrogen gas blowing port, and the mixture was allowed to react at 85°C. At a time point at which the proportion of residual isocyanate groups reached 4.0%, the temperature was decreased to 70°C, and 2.7 g of 2-hydroxyethyl acrylate (0.023 moles, manufactured by Tokyo Chemical Industry Co., Ltd.) was added thereto. The reaction was completed at a time point at which the proportion of residual isocyanate groups reached 0.2%. After the completion, 0.01 g of para-methoxyphenol (manufactured by Tokyo Chemical Industry Co., Ltd.) was added thereto as a polymerization inhibitor, and then the solvent was distilled off under reduced pressure. Thus, 204 g of polyurethane acrylate A-1 was obtained. The weight average molecular weight of the polyurethane acrylate A-1 measured by gel permeation chromatography was 5,000.

The compounds of A-2 to A-21 were obtained by the same method as that of Synthesis Example 1, except that the raw materials and the amounts of use thereof were appropriately changed.

### [Production of artificial nail composition]

The various components indicated in the following tables were uniformly mixed, and artificial nail compositions G-1 to G-43 were obtained. Meanwhile, the values shown in the tables represent parts by mass, and the symbol "-" means that the relevant component is not added.

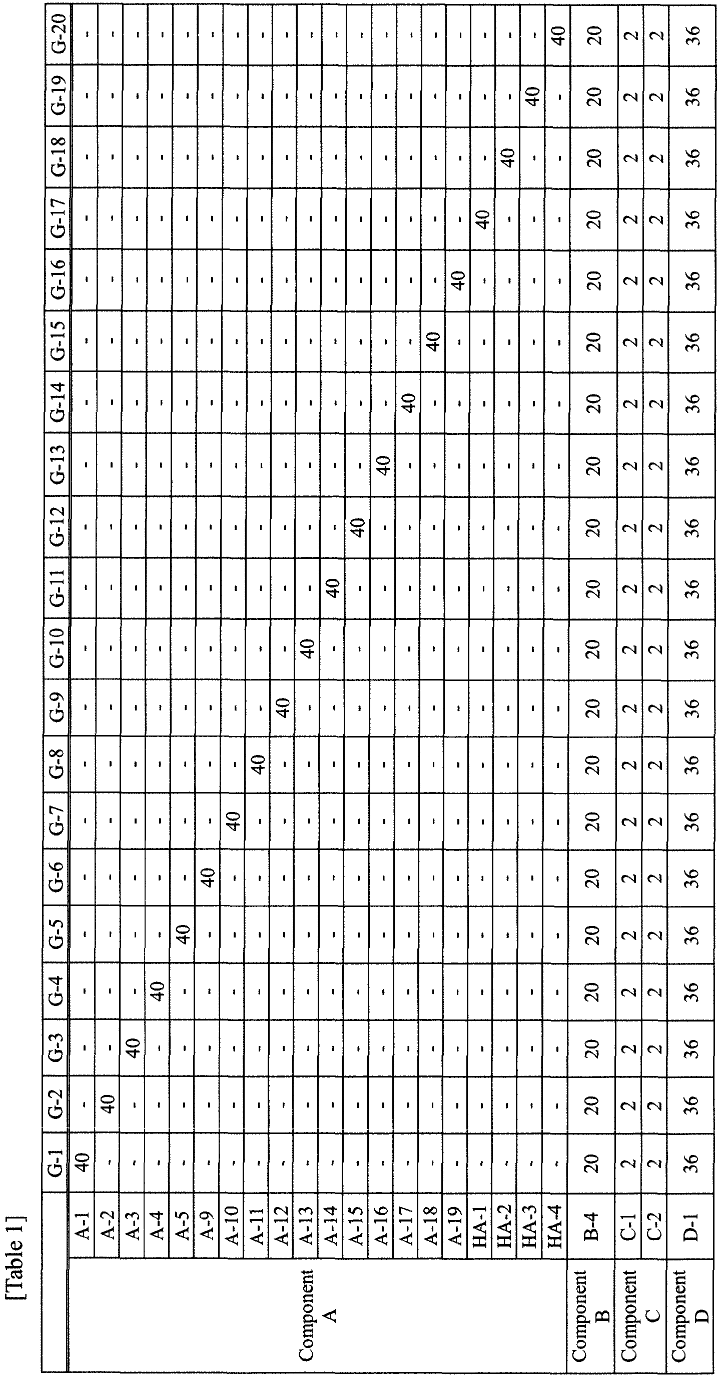

**[Table 2]**

| | | G-21 | G-22 | G-23 | G-24 | G-25 | G-26 | G-27 | G-28 | G-29 | G-30 | G-31 | G-32 | G-33 | G-34 |
|---|---|---|---|---|---|---|---|---|---|---|---|---|---|---|---|
| Component A | A-1 | 40 | 40 | 40 | 40 | 40 | 40 | 40 | 40 | 40 | 40 | 50 | 50 | 40 | 50 |
| Component B | B-1 | - | - | - | - | - | 20 | - | - | - | - | - | - | - | - |
| | B-2 | - | - | - | - | - | - | 20 | - | - | - | - | - | - | - |
| | B-3 | - | - | - | - | - | - | - | 20 | - | - | - | - | - | - |
| | B-4 | 20 | 20 | 20 | 20 | 20 | - | - | - | 20 | 20 | - | - | 20 | 5 |
| Component C | C-1 | 2 | 2 | 2 | 2 | 2 | 2 | 2 | 2 | 2 | 2 | 2 | 2 | - | 2 |
| | C-2 | 2 | 2 | 2 | 2 | 2 | 2 | 2 | 2 | 2 | 2 | 2 | 2 | - | 2 |
| | C-3 | - | - | - | - | - | - | - | - | - | - | - | - | 4 | - |
| Component D | D-1 | - | - | - | - | - | 18 | 36 | 36 | 36 | 3 | 46 | - | 36 | 41 |
| | D-2 | 36 | - | - | - | - | - | - | - | - | - | - | - | - | - |
| | D-3 | - | 36 | - | - | - | - | - | - | - | - | - | - | - | - |
| | D-4 | - | - | 36 | - | - | - | - | - | - | - | - | - | - | - |
| | D-5 | - | - | - | 36 | 18 | - | - | - | - | 33 | - | 46 | - | |
| | D-6 | - | - | - | - | 18 | - | - | - | - | - | - | - | - | - |
| | D-7 | - | - | - | - | - | 18 | - | - | - | - | - | - | - | - |

**[Table 3]**

| | | G-35 | G-36 | G-37 | G-38 | G-39 | G-40 | G-41 | G-42 | G-43 |
|---|---|---|---|---|---|---|---|---|---|---|
| Component A | A-1 | 35 | - | - | - | - | 35 | 35 | 35 | 45 |
| | HA-1 | - | 35 | - | - | - | - | - | - | - |
| | HA-2 | - | - | 35 | - | - | - | - | - | - |
| | HA-3 | - | - | - | 35 | - | - | - | - | - |
| | HA-4 | - | - | - | - | 35 | - | - | - | - |
| Organic red pigment | | 5 | 5 | 5 | 5 | 5 | 5 | 5 | 5 | 5 |
| Component B | B-4 | 20 | 20 | 20 | 20 | 20 | 20 | 20 | 20 | - |
| Component C | C-1 | 2 | 2 | 2 | 2 | 2 | 2 | 2 | 2 | 2 |
| | C-2 | 2 | 2 | 2 | 2 | 2 | 2 | 2 | 2 | 2 |
| Component D | D-1 | 36 | 36 | 36 | 36 | 36 | - | - | - | 46 |
| | D-2 | - | - | - | - | - | 36 | - | - | - |
| | D-3 | - | - | - | - | - | - | - | - | - |
| | D-4 | - | - | - | - | - | - | - | - | - |
| | D-5 | - | - | - | - | - | - | 36 | 18 | - |
| | D-6 | - | - | - | - | - | - | - | 18 | - |

The component A to the component D used were as follows.
(Component A)
   · HA-1 (Comparative Example): Pulple light UV-3000B (ester type polyurethane acrylate, manufactured by Nippon Synthetic Chemical Industry Co., Ltd., Mw = 18,000, number of functional groups: 2)
   · HA-2 (Comparative Example): Pulple light UV-3300B (ether type polyurethane acrylate, manufactured by Nippon Synthetic Chemical Industry Co., Ltd., Mw = 13,000, number of functional groups: 2)
   · HA-3 (Comparative Example): Pulple light UV-3700B (ether type polyurethane acrylate, manufactured by Nippon Synthetic Chemical Industry Co., Ltd., Mw = 38,000, number of functional groups: 2)
   · HA-4 (Comparative Example): Pulple light UV-3200B (ester type polyurethane acrylate, manufactured by Nippon Synthetic Chemical Industry Co., Ltd., Mw = 10,000, number of functional groups: 2)
(Component B)
   · B-1: Polyurethane described below (Mw (weight average molecular weight): 20,000, the numerical value in the structure represents the molar ratio) 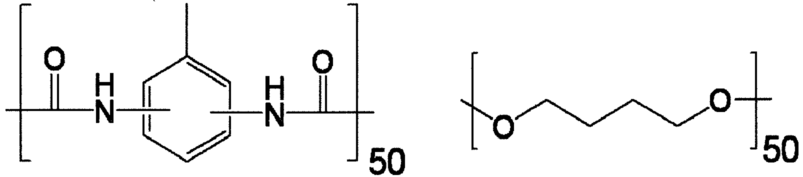
   B-2: Polymethyl methacrylate (Mw: 20,000)
   · B-3: Polyurethane described below (Mw: 50,000, the numerical value in the structure represents the molar ratio) 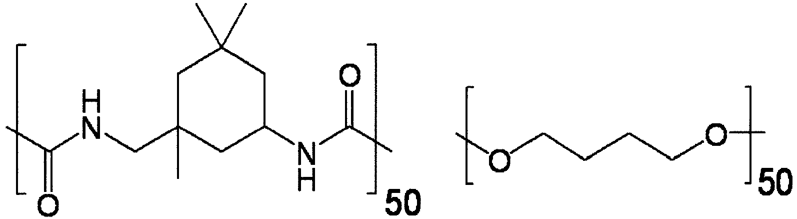
   B-4: Polyurethane described below (Mw: 20,000, the numerical value in the structure represents the molar ratio) 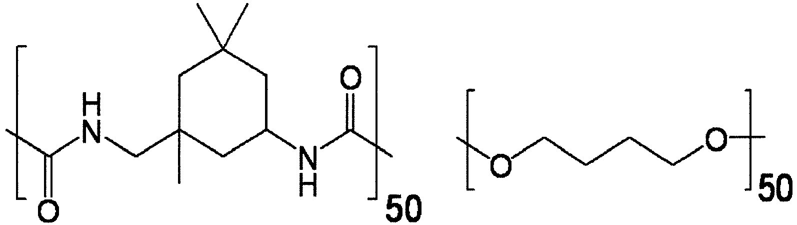
(Component C)
   · C-1: IRGACURE 184 (1-hydroxycyclohexyl phenyl ketone, acetophenone compound, manufactured by BASF SE)
   C-2: IRGACURE TPO (2,4,6-trimethylbenzoyl diphenylphosphine oxide, acylphosphine oxide compound, manufactured by BASF SE)
   C-3: IRGACURE 784 (bis(η5-2,4-cyclopentadien-1-yl)-bis(2,6-difluoro-3-(1H-pyrrol-1-yl)-phenyl)titanium, titanocene-based compound, manufactured by BASF SE)
(Component D)
   - D-1: Hydroxyethyl methacrylate (manufactured by Tokyo Chemical Industry Co., Ltd.)
   - D-2: Hydroxypropyl methacrylate (manufactured by Kyoeisha Chemical Co., Ltd.)
   - D-3: Methacrylic acid (manufactured by Tokyo Chemical Industry Co., Ltd.)
   - D-4: Acrylic acid (manufactured by Tokyo Chemical Industry Co., Ltd.)
   - D-5: Methyl methacrylate (manufactured by Tokyo Chemical Industry Co., Ltd.)
   - D-6: Isobornyl methacrylate (manufactured by Tokyo Chemical Industry Co., Ltd.)
   - D-7: Urethane dimethacrylate represented by the following formula:

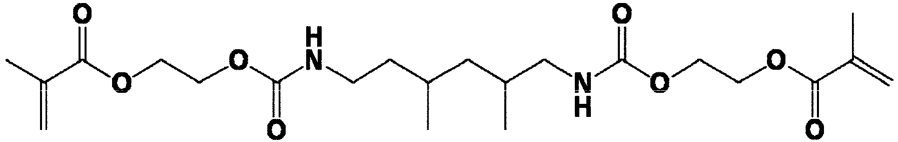

Meanwhile, for D-7, a compound synthesized by reacting 1 molar equivalent of a corresponding diisocyanate compound with 2 molar equivalents of 2-hydroxyethyl methacrylate was used.

### (Formation of artificial nail)

### <Formation Example 1>

0.25 g of an artificial nail composition thus obtained was applied on fingernails using a paint brush, and the artificial nail composition was irradiated with an ultraviolet lamp (36 W) for 2 minutes. Thereafter, the surface was cleaned with ethanol, and then the artificial nails thus formed were observed by visual inspection. The artificial nails were in a completely solidified state. The film thicknesses of the artificial nails at this time were measured, and it was found that the thicknesses were about 300 µm (± 10 µm),

### <Formation Example 2>

On artificial nails having a thickness of about 300 µm (± 10 µm) formed from a commercially available base gel (manufactured by Moga Brook Co., Ltd.) by the same method as Formation Example 1, the artificial nail composition was applied as a color layer with a paint brush, and was irradiated with an ultraviolet lamp (36 W) for 2 minutes. Uncured portions were wiped out with ethanol, and then a commercially available top gel (manufactured by Moga Brook Co., Ltd.) was applied thereon as a top layer using a paint brush and was similarly irradiated with an ultraviolet lamp (36 W) for 2 minutes. The artificial nails thus formed were observed by visual inspection, and the artificial nails were in a completely solidified state. The film thickness combining the color layer and the top layer was about 900 µm.

### (Evaluation of artificial nails)

### <Gloss>

The gloss of an artificial nail after the formation was evaluated by visual inspection under fluorescent lamp lighting according to the following evaluation criteria.

### - Evaluation criteria -

A: The fluorescent lamp is reflected on the surface of the nail, and the image can be clearly recognized.
B: The fluorescent lamp is reflected on the surface of the nail, and the image can be recognized slightly clearly.
C: The fluorescent lamp is reflected on the surface of the nail, but the image cannot be clearly recognized.
D: The fluorescent lamp is not reflected on the surface of the nail.

### <Removability>

The surface and the tip of an artificial nail thus obtained were scratched with a file (nail file). Thereafter, a cotton ball soaked with acetone was wound around the artificial nail, and the artificial nail was wrapped with aluminum foil. After 2 minutes passed, the cotton ball was removed, and the acetone-immersed part was subjected to a detachment operation using a wooden spatula, and the ease of removal from the substrate (nail) was evaluated according to the following evaluation criteria.

### - Evaluation criteria -

A: The artificial nail could be easily removed.
B: The artificial nail could be removed.
C: The artificial nail could be removed, but a small amount of residual film was left, and the residual film was wiped out using a removal liquid.
D: The artificial nail could not be removed.

### <Adhesiveness>

The uppermost layer of an artificial nail thus obtained was scraped using a pencil having the HB hardness with a certain load, and the presence or absence of detachment and the presence or absence of scratching were evaluated according to the following evaluation criteria.

### - Evaluation criteria -

A: Both scratching and detachment did not occur.
B: Detachment did not occur, but slight scratching occurred.
C: Detachment did not occur, but scratching occurred.
D: Detachment occurred.

### <Adhesiveness in warm water - 1>

An artificial nail thus obtained was immersed in tap water (pH 6.4) at 45°C, and after 10 minutes passed, the uppermost layer of the artificial nail thus obtained was scraped using a pencil having the HB hardness with a certain load, and the presence or absence of detachment and the presence or absence of scratching were evaluated according to the following evaluation criteria.
A: Both scratching and detachment did not occur.
B: Detachment did not occur, but slight scratching occurred.
C: Detachment did not occur, but scratching occurred.
D: Detachment occurred.

### <Adhesiveness in warm water - 2>

An artificial nail thus obtained was immersed in a water tank containing tap water (pH 6.4) at 45°C, and an operation of pressing the artificial nail against a water tank wall to the extent that the nail was pressed and bent was repeated 100 times.
A: Detachment did not occur.
B: Detachment occurred in the periphery of the artificial nail composition.
C: Detachment occurred to the interior of the artificial nail composition.
D: Detachment occurred, and the artificial nail was removed from the nail.

### (Examples 1 to 28 and Comparative Examples 1 to 6)

For artificial nails produced according to Formation Example 1, gloss, adhesiveness, adhesiveness in warm water -1 and -2, and removability were evaluated. The results are presented in the table.

**[Table 4]**

| | Artificial nail composition | Formation Example | Gloss | Adhesiveness | Adhesiveness in warm water -1 | Adhesiveness in warm water -2 | Removability |
|---|---|---|---|---|---|---|---|
| Example 1 | G-1 | 1 | A | A | A | A | A |
| Example 2 | G-2 | 1 | A | A | A | B | A |
| Example 3 | G-3 | 1 | A | A | A | B | A |
| Example 4 | G-4 | 1 | A | A | A | B | A |
| Example 5 | G-5 | 1 | A | A | A | B | A |
| Example 6 | G-6 | 1 | A | A | A | A | A |
| Example 7 | G-7 | 1 | A | A | A | B | A |
| Example 8 | G-8 | 1 | A | A | A | B | A |
| Example 9 | G-9 | 1 | B | B | A | A | B |
| Example 10 | G-10 | 1 | B | B | A | A | B |
| Example 11 | G-11 | 1 | B | B | A | A | B |
| Example 12 | G-12 | 1 | B | B | A | A | B |
| Example 13 | G-13 | 1 | B | B | A | A | B |
| Example 14 | G-14 | 1 | B | B | A | A | B |
| Example 15 | G-15 | 1 | B | B | A | B | B |
| Example 16 | G-16 | 1 | B | A | A | B | A |
| Example 17 | G-21 | 1 | B | A | A | A | B |
| Example 18 | G-22 | 1 | B | A | A | A | B |
| Example 19 | G-23 | 1 | B | A | A | A | B |
| Example 20 | G-26 | 1 | B | A | B | B | B |
| Example 21 | G-27 | 1 | B | A | A | A | B |
| Example 22 | G-28 | 1 | B | B | A | A | B |
| Example 23 | G-29 | 1 | B | A | A | A | c |
| Example 24 | G-30 | 1 | B | B | B | B | B |
| Example 25 | G-33 | 1 | B | B | B | B | C |
| Example 26 | G-24 | 1 | B | B | B | C | C |
| Example 27 | G-25 | 1 | B | B | C | C | C |
| Example 28 | G-34 | 1 | A | A | B | B | B |
| Comparative Example 1 | G-17 | 1 | B | B | D | C | C |
| Comparative Example 2 | G-18 | 1 | B | B | D | D | A |
| Comparative Example 3 | G-19 | 1 | B | B | D | D | A |
| Comparative Example 4 | G-20 | 1 | B | B | D | C | C |
| Comparative Example 5 | G-31 | 1 | C | D | D | D | C |
| Comparative Example 6 | G-32 | 1 | C | D | D | D | C |

It can be seen from the results of the table that the artificial nails obtained from the artificial nail compositions of the invention is well balanced between high adhesiveness and removability. The artificial nail exhibits suitable behavior particularly in connection with deformation in warm water, and thus it can be seen that the artificial nail has high adhesiveness.

### (Examples 29 to 32 and Comparative Examples 7 to 11)

For artificial nails produced according to Formation Example 2, gloss, adhesiveness, adhesiveness in warm water -1 and -2, and removability were evaluated. The results are presented in the table.

**[Table 5]**

| | Artificial nail composition | Formation Example | Gloss | Adhesiveness | Adhesiveness in warm water -1 | Adhesiveness in warm water -2 | Removability |
|---|---|---|---|---|---|---|---|
| Example 29 | G-35 | 2 | A | A | A | A | A |
| Example 30 | G-40 | 2 | B | A | A | A | B |
| Example 31 | G-41 | 2 | B | B | B | C | C |
| Example 32 | G-42 | 2 | B | B | C | C | C |
| Comparative Example 7 | G-36 | 2 | B | B | D | C | C |
| Comparative Example 8 | G-37 | 2 | B | B | D | D | A |
| Comparative Example 9 | G-38 | 2 | B | B | D | D | A |
| Comparative Example 10 | G-39 | 2 | B | B | D | C | C |
| Comparative Example 11 | G-43 | 2 | B | C | D | D | C |

It can be seen from the results of the table that the artificial nails obtained from the artificial nail compositions of the invention are well balanced between high adhesiveness and removability. The artificial nail exhibits suitable behavior particularly in connection with deformation in warm water, and thus it can be seen that the artificial nail has high adhesiveness. Also, it is shown that these performances are manifested without any problem even if the artificial nail is made to function as a color layer by adding a colorant to the artificial nail composition.

## Claims

1. An artificial nail composition comprising:
a polyurethane (meth)acrylate having a polycarbonate structure as component A;
a binder polymer having a weight average molecular weight of 10,000 or more as component B; and
a photopolymerization initiator as component C.

2. The artificial nail composition according to claim 1, wherein the component A is represented by the following Formula 1: 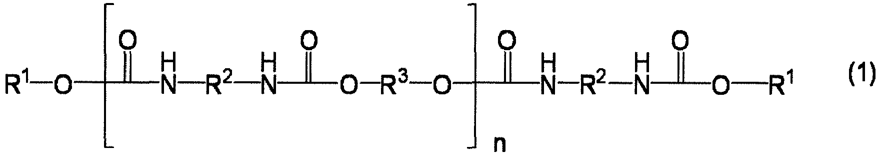 wherein in Formula 1, R¹'s each independently represent a monovalent group having a (meth)acryloyloxy group at an end; R² and R³ each independently represent a divalent organic group; at least one R³ represents a group represented by the following Formula 2; and n represents an integer of 1 or more, and 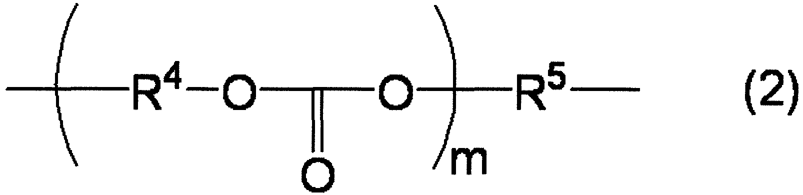 wherein in Formula 2, R⁴ and R⁵ each independently represent a divalent organic group; and m represents an integer of 1 or more.

3. The artificial nail composition according to claim 1 or 2, wherein the weight average molecular weight of the component A is 1,000 or more.

4. The artificial nail composition according to any one of claims 1 to 3, wherein the artificial nail composition comprises the component B at a proportion of 15% by mass or more relative to the total amount of the artificial nail composition.

5. The artificial nail composition according to any one of claims 1 to 4, wherein the artificial nail composition comprises an acetophenone compound and/or an acylphosphine oxide compound as the component C.

6. The artificial nail composition according to any one of claims 1 to 5, further comprising: a polymerizable compound as component D.

7. The artificial nail composition according to claim 6, wherein the component D includes an ethylenically unsaturated compound.

8. The artificial nail composition according to claim 6 or 7, wherein the component D includes a monofunctional ethylenically unsaturated compound.

9. The artificial nail composition according to any one of claims 6 to 8, wherein the component D includes at least one compound selected from the group consisting of a (meth)acrylate compound having a hydroxy group, a (meth)acrylate compound having a carboxy group, and (meth)acrylic acid.

10. The artificial nail composition according to any one of claims 6 to 9, wherein the component D includes at least one compound selected from the group consisting of hydroxyethyl (meth)acrylate, hydroxypropyl (meth)acrylate, hydroxybutyl (meth)acrylate, a polyalkylene glycol mono(meth)acrylate, and (meth)acrylic acid.

11. The artificial nail composition according to any one of claims 6 to 10, wherein the component D includes at least one compound selected from the group consisting of hydroxyethyl (meth)acrylate, hydroxypropyl (meth)acrylate, and (meth)acrylic acid.

12. The artificial nail composition according to any one of claims 6 to 11, wherein the component D includes at least hydroxyethyl (meth)acrylate.

13. An artificial nail comprising a layer formed by exposing the artificial nail composition according to any one of claims 1 to 12 to light.

14. A method for forming an artificial nail, the method comprising:
a step of applying the artificial nail composition according to any one of claims 1 to 12 on a nail of a human being or an animal, or on another artificial nail, and forming a coating film thereon; and
a step of exposing the coating film to light, and thereby forming an artificial nail.

15. A nail art kit comprising the artificial nail composition according to any one of claims 1 to 12.
